# EUROPEAN PATENT SPECIFICATION

(11) **EP 0 740 965 B1**
(45) Date of publication and mention of the grant of the patent: **14.06.2000**
(21) Application number: 95115643.9
(22) Date of filing: 04.10.1995
(51) Int. Cl.: B05C 1/16, B05C 1/12

(54) **Process and apparatus for the manufacture of a web for use as a coversheet in the production of sanitary disposable absorbent products**
Verfahren und Vorrichtung zur Herstellung einer Bahn zur Anwendung als Decklage in der Herstellung von sanitären absorbierenden Wegwerfartikeln
Procédé et appareil pour la production d'une bande pour servir de feuille de couverture dans la fabrication de produits sanitaires absorbants jetables

(30) Priority: 05.05.1995 US 434672
(43) Date of publication of application: 06.11.1996
(73) Proprietor: AVGOL LTD., IL-58101 Holon (IL)
(72) Inventor: Goldwasser, Moshe D., Tel Aviv 69409 (IL)
(74) Representative: Finck, Dieter, Dr.Ing.

(56) References cited:
- EP-A- 0 611 607
- CH-A- 669 915
- FR-A- 1 557 477
- US-A- 3 032 006

## Description

The invention relates to a process for the manufacture of a web for use as a coversheet in the production of sanitary disposable absorbent products such as disposable baby diapers, sanitary napkins, adult incontinence pads, and hospital bed pads, which process includes supplying a web of pre-formed, well-integrated nonwoven or woven material, providing a supply of fluidic material limited to a substance which alters the fluid-transmissible characteristic of the web, providing an applicator roll partially submerged in said supply of said fluidic material and rotating said applicator roll so that the fluidic material is deposited on the surface of said applicator roll and carried thereby as the web rotates.

The invention relates further to an apparatus for coating a web of integrated material particularly for carrying out the above method, which apparatus comprises an applicator roll, a body of fluidic material, and means to rotate said applicator roll partially submerged in said body of fluidic material whereby to coat said surface of said applicator roll with said fluidic material.

Such a process and apparatus are disclosed by EP 0 611 607 A1, are considered as the pertinent prior art, and are described below in detail.

It is well-known in the art for the manufacture of disposable baby diapers or other sanitary disposable absorbent products such as sanitary napkins, adult incontinence pads, and hospital bed pads to provide a structure wherein the coversheet or coverstock or top sheet, i.e. that portion of the product which is in contact with the patient's skin, is made of hydrophyllic material so as to be pervious to liquids such as urine. This permits ready pass-through of the liquid into the absorbent core or the pad which lies beneath the coverstock. In the past, some of the coverstocks have also been made of cotton, or blends of cotton and rayon, or blends of rayon with a bonding fiber such as polyethylene.

Such coverstock, however, has been undesirable because it tends to retain the moisture and thus feel wet to touch and because it keeps the skin wet and is more likely to cause skin rash or diaper rash or the like.

Therefore, in more recent years, it has been found desirable to use a coverstock made of hydrophobic fibers or filaments, such as polypropylene or polyester, either carded, spun-bonded or melt-blown, or the like, but because such material inherently tends to interfere with the pass-through of urine into the absorbent pad, it has been found necessary to treat the web with a hydrophyllicity-inducing material such as a surfactant (Triton X-102 distributed by Rohm & Haas Co. of Philadelphia, Pennsylvania, or MAGNASOFT manufactured by the Union Carbide Company).

The surfactant usually is incorporated with the fibers by the fiber manufacturers before being sent to the web manufacturer, who form a web which is substantially uniformly hydrophyllic. A web thus formed when used as the top sheet in a baby or adult diaper is then coated in areas where perviousness is not only unnecessary but also undesirable. In a more recent form of diaper construction, a strip of nonwoven web of hydrophyllic material is assembled side-by-side between two strips or webs of hydrophobic fibers. When such a 3-strip web is placed upon a diaper, with the-pervious strip in the longitudinal center of the diaper, the urine can pass to the absorbent core through the center strip but not along the sides where the coverstock is impervious. However, such a pre-formed web assembled from three different materials is costly to make and more difficult to run on a diaper machine because of the seamlines between the hydrophyllic and hydrophobic strips.

The pertinent prior art according to EP 0 611 607 A1 is described with the aid of drawings in which
- Fig. 1: schematically shows an apparatus for the production of a web,
- Fig. 2: represents a reproduction of the surface of a hydrophobic web material which has areas treated to render it hydrophyllic, showing how the hydrophobic areas repel liquid while the hydrophyllic areas permit the liquid to pass through,
- Fig. 3: perspectively shows an applicator roll used in the apparatus of Fig. 1,
- Fig. 4: is a plan view of a baby diaper with a full length strip of pervious area in the center of the topsheets made with the apparatus of Fig. 1, and
- Fig. 5: shows as Fig. 4 the diaper, but with an intermittent strip produced with the applicator roll of Fig. 3.

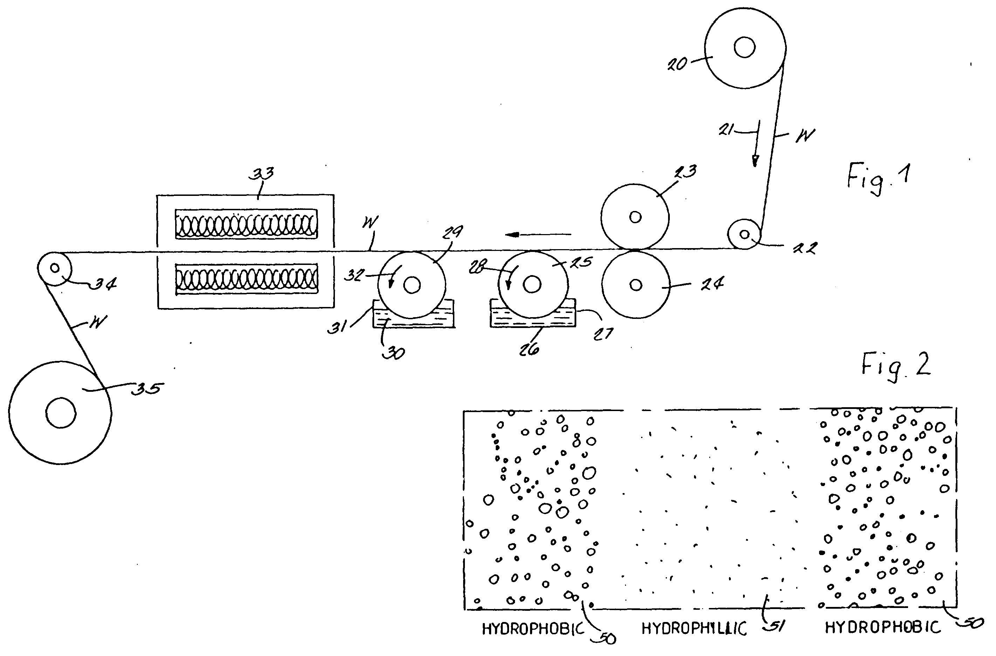

Fig. 1 shows a pre-formed, well-integrated roll 20 of a web W of a nonwoven (or woven) coverstock material which is unwound in the direction of the arrow 21, turning around a carrier roll 22 so that it can pass between rolls 23 and 24 which comprise a set of press or calender rolls.

Thereafter the web W passes over a first "kiss" or "padding" roll 25 which applies a selected material 26 from a pan or holder 27 when the roll 25 rotates within the material 26 in the direction of the arrow 28. The material 26 may be either a surfactant, if the web W is hydrophobic in character, or a material, which induces hydrophobicity, if the web is hydrophyllic.

If desirable, a second roll 29 can be used, also rotating in a bath 30 of material held within a pan 31 and rotating in the direction of the arrow 32 to apply a second coating. This second coating may be of the same material as applied in the first "kiss" roll or it may be opposite in character and applied in a different area of the web W.

Thereafter, the web W passes through a drying chamber 33 and around a turning roll 34 to be rolled up on a winder 35. The winder 35 may be 3,05 m (120 inches) wide and may be a combination winder/slitter so that individual rolls of finished coverstock of appropriate width may be produced. Desirable widths presently used in the industry are 38 cm (15 inches), and on such 38 cm (15 inch) wide webs the central pervious strip may be 13 cm (5 inches) wide with a 13 cm (5 inch) wide strip of impervious material on each side of the central strip.

The application of the material may be continuous so as to provide an uninterrupted treated area, as shown in Fig. 4. This "zebra-like" pattern may be preferred for a variety of reasons, not the least significant of which is ease and economy of operation, even though a small amount of material may be applied in areas where its presence is not critical to the operation of the finished product.

Downstream of the dryer member 33, either between the dryer 33 and the turning wheel 34 or between the turning wheel 34 and the windup roll 35, the web W may be slit by appropriate slitter knives (not shown) and thus wound up in narrow "doughnut-size" rolls for shipment to the customer.

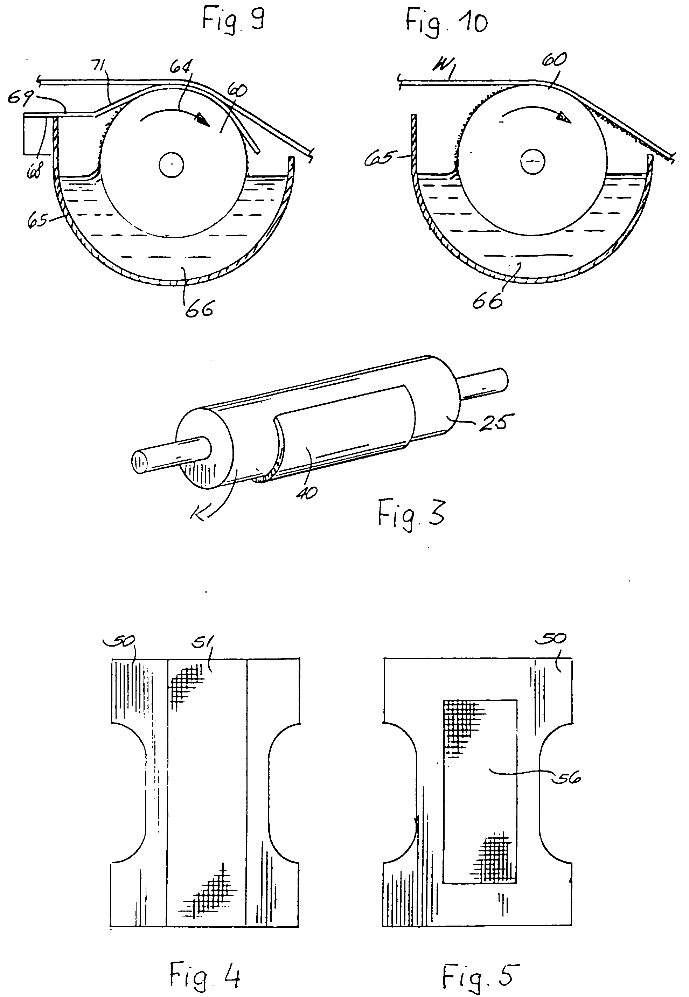

The applicator roll 25 of Fig. 1 may be a "kiss" roll K, as shown in Fig. 3, which has a raised portion 40 which picks up the material 26 from the carrier pan 27 or 31. The shape of the raised portion 40 may be chosen to provide the desired area of deposition.

Fig. 5 illustrates how the coating material 56 can be limited to a rectangular area which stops short of the ends of the diaper.

Although a primary desire is to create a hydrophobic web material treated with a surfactant to render treated areas 51, 56 hydrophyllic, it is to be understood that the reverse is just as possible, namely, that the web can be hydrophyllic in nature and the treated areas 51, 56 can be rendered hydrophobic.

As few or as many of the kiss rolls may be utilized and they do not all have to be of the same width. Thus the dimensions of the pervious portion 51 in the final web may be of a dimension as desired by the customer, and the impervious or hydrophobic portions 50 can also be selected to the customer's preference. However, for every width a separate "kiss" roll is to be provided and mounted.

Therefore, it is the object of the invention to provide an apparatus and process as mentioned above in connection with EP 0 611 607 A1, the latter modifying the liquid-pervious characteristics of a wide web of material in a plurality of selected relatively narrow zones, and the former allowing easy adjustment to change the width of the relatively pervious zone.

This object is achieved with the process as mentioned above in connection with EP 0 611 607 A1 by providing at least one shutter which is positioned above said applicator roll, and by moving said web in contact with said shutter and an un-submerged portion of said applicator roll having fluidic material thereon so that said web contacts only those areas of the applicator roll not covered by said shutter to pick up the fluidic material from the applicator roll.

Preferably the linear speed of said web is not greater than 10% of the peripheral speed of said applicator roll.

It is convenient that the web is made of hydrophobic material and the fluidic material is such as to change the characteristic of the web material, in an area where the web contacts the applicator roll, from hydrophobic to hydrophyllic.

Advantageously the web is spun-bonded, melt-blown, thermo-bonded or adhesively bonded prior to application of the fluidic material.

The above object is achieved with the apparatus as mentioned above in connection with EP 0 611 607 A1 comprising at least one shutter having an upper surface and positioned above the un-submerged surface of said applicator roll and means to guide said web across the upper surface of the shutter and in contact with the un-submerged portion of said applicator roll not covered by said shutter, whereby to apply said fluidic material only to the portion of the web not having a shutter above the applicator roll.

Preferably, a tensioning roll is provided adjacent said applicator roll for controlling tension in the web as well as to control the contact area of said web with said applicator roll.

Usually there are means for drying the web and fluidic material after passing from the applicator roll.

Preferred are also means for controlling the speed of the web and the speed of rotation of the applicator roll so that the linear speed of movement of the web is not the same as the peripheral speed of the surface of the applicator roll.

The speed controlling means can insure that the peripheral speed of the applicator roll is at least 10 times greater than the linear speed of the web.

With the present invention the zones to which liquid is applied to can be easily selected and adjusted maintaining the usual cylindric applicator rolls of the prior art in site. According to the invention coating selective zones of thin webs to change the pervious character thereof can be easily achieved by technically simple means. With the invention a well-integrated woven or non-woven web of hydrophobic fibers can be treated to make selective areas hydrophyllic. It also can be used to make a web of hydrophyllic fibers selectively hydrophobic. The web may be non-woven, its fibers are hydrophobic in nature, e.g. dry-laid or melt-blown polypropylene or polyethylene fibers or spun-bonded hydrophobic filaments. A woven web made of cotton or other hydrophyllic fibers may also be used if the end result is to create partially hydrophobic areas on a hydrophyllic web. The areas of liquid are positioned on the web only where desired so as to eliminate the excessive cost of unwanted and unnecessary coating material.

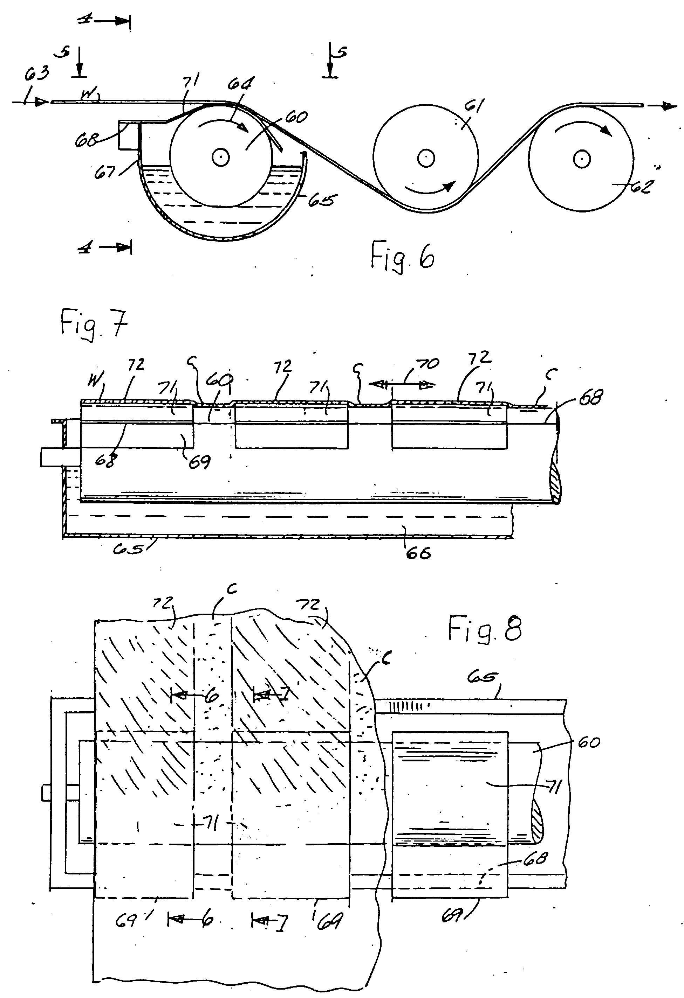

Embodiments of the invention are further described in detail with reference to the drawings in which
- Fig. 6: schematically shows the means of the present invention for modifying the liquid-pervious characteristics of a web in a plurality of easily selectable zones,
- Fig. 7: is a cross-sectional view taken along line 4-4 of Fig. 6,
- Fig. 8: is a top plan view of the applicator roll according to the invention taken generally along lines 5-5 of Fig. 6,
- Fig. 9: is a cross-sectional view taken generally along line 6-6 of Fig. 8, and
- Fig. 10: is a cross-sectional view taken along line 7-7 of Fig. 8.

The applicator rolls 60 and the rolls 61 and 62 of Fig. 6 to 10 replace the applicator rolls 25 and 29 in the prior art apparatus of Fig. 1. The web W moving in the direction of the arrow 63 passes over the top of said applicator roll 60 beneath said roll 61 and over the top of said roll 62.

Said applicator roll 60 rotates in the direction of arrow 64, partly submerged in a pan 65 containing a fluidic material, in this case a liquid surfactant 66.

As the applicator roll 60 rotates, it picks up on the surface thereof the liquid surfactant 66 which is carried out of the pan 65 and deposited against the web W in the areas C where the web W comes into contact with the surface of the applicator roll 60. Thus the applicator roll 60 carries a measured quantity of liquid surfactant 66 onto the web W. As the web W passes from said applicator roll 60 beneath said roll 61 to the top of said roll 62 the web W is under tension and the pressure of the roll 61 against the web W insures migration of the liquid into the web W.

The roll 61 is a tensioning roll which keeps the web W stretched between the applicator roll 60 and the roll 62 and tightly against the top of the applicator roll 60. The tension can be changed, as desired, by moving the roll 61 up or down in the direction of arrow A. The position of the roll 61 also determines how much surface of the applicator roll 60 and of the rolls 61 and 62 is in contact with the web W, thus also insuring control of the migration of the liquid surfactant 66 into the web W.

After the impregnated web W passes from the roll 62, it moves into drying chambers 33 (Fig. 1).

As can be seen particularly in Fig. 6 and 7, at the upstream side 67 of the pan 65, there are supported on the upper edge 68 thereof a plurality of movable covers or shutters 69. These shutters 69 are mounted on the edge 68 in such a way that they can be moved along the edge 68 in the direction of the arrow 70 shown more particularly in Fig. 7 and 8.

Each shutter 69 has a flexible downstream portion 71 which lies draped on top of the surface of the applicator roll 60 and prevents the liquid surfactant 66 from coming into contact with the web W at those portions where the flexible portion 71 is between the web W and the surface of the applicator roll 60, which has the film of liquid thereon.

In the area C between the shutters 69, the web W is pulled into contact with the surface of the roll 60 by the tension which is created in the web W by the tensioning roll 61 and thus in those areas C, between the shutters 69, the web W is impregnated with liquid surfactant 66 or other fluidic material.

It is to be easily understood that the shutters 69 can be of a specific width and particularly dimensioned so that the uncoated web portions 72 may be as wide as desired, and the placement of the shutters 69, by sliding them along the upper edge 68 of the pan portion 67, determines the width of the impregnated portions C between the unimpregnated portions 72.

## Claims

1. Process for the manufacture of a web for use as a coversheet in the production of sanitary disposable absorbent products such as disposable baby diapers, sanitary napkins, adult incontinence pads, and hospital bed pads, which includes:
- supplying a web (W) of pre-formed, well-integrated nonwoven or woven material,
- providing a supply of fluidic material (66), limited to a substance which alters the fluid-transmissible characteristic of the web (W),
- providing an applicator roll (60) partially submerged in said supply of said fluidic material (66) and rotating said applicator roll (60) so that the fluidic material (66) is deposited on the surface of said applicator roll (60) and carried thereby as the web (W) rotates,
characterized by
- providing at least one shutter (69) which is positioned above said applicator roll (60), and
- moving said web (W) in contact with said shutter (69) and an un-submerged portion of said applicator roll (60) having fluidic material (66) thereon so that said web (W) contacts only those areas (C) of the applicator roll (60) not covered by said shutter (69) to pick up the fluidic material (66) from the applicator roll (60).

2. Process according to Claim 1, characterized in that the linear speed of said web (W) is not greater than 10% of the peripheral speed of said applicator roll (60).

3. Process according to Claim 1 or 2, characterized in that the web (W) is made of hydrophobic material and the fluidic material (66) is such as to change the characteristic of the web material, in an area (C) where the web (W) contacts the applicator roll (60), from hydrophobic to hydrophyllic.

4. Process according to Claim 3, characterized in that the web (W) is spun-bonded, melt-blown, thermo-bonded or adhesively bonded prior to application of the fluidic material (66).

5. Apparatus for coating a web of integrated material, particularly for carrying out the method according to one of the Claims 1 to 4, comprising
- an applicator roll (60),
- a body of fluidic material (66), and
- means to rotate said applicator roll (60) partially submerged in said body of fluidic material (66) whereby to coat said surface of said applicator roll (60) with said fluidic material (66),
characterized by
- at least one shutter (69) having an upper surface and positioned above the un-submerged surface of said applicator roll (60), and
- means to guide said web (W) across the upper surface of the shutter (69) and in contact with the un-submerged portion of said applicator roll (60) not covered by said shutter (69), whereby to apply said fluidic material (66) only to the portion (C) of the web (W) not having a shutter (69) above the applicator roll (60).

6. Apparatus according to Claim 5, characterized by a tensioning roll (61) adjacent said applicator roll (60) for controlling tension in the web (W) as well as to control the contact area (C) of said web (W) with said roll (61) and said applicator roll (60).

7. Apparatus according to Claim 5 or 6, characterized by means for drying the web (W) and fluidic material (66) after passing from the applicator roll (60).

8. Apparatus according to one of the Claims 5 to 7, characterized by means for controlling the speed of the web (W) and the speed of rotation of the applicator roll (60) so that the linear speed of movement of the web (W) is not the same as the peripheral speed of the surface of the applicator roll (60).

9. Apparatus according to Claim 8, characterized in that the speed controlling means insures that peripheral speed of the applicator roll (60) is at least 10 times greater than the linear speed of the web (W).

## Patentansprüche

1. Verfahren zur Herstellung einer Bahn zur Verwendung als Deckschicht bei der Produktion von wegwerfbaren sanitären absorbierenden Erzeugnissen, wie Einweg-Babywindeln, Monatsbinden, Inkontinenzpolstern für Erwachsene sowie Hospitalbettenpolster, bei welchen
- eine Bahn (W) eines vorgeformten, gut integrierten Vlies- oder Gewebematerials zugeführt wird,
- ein Vorrat an fluidischem Material (66) bereitgestellt wird, das auf eine Substanz begrenzt ist, welche die Fluidübertragbarkeitseigenschaft der Bahn (W) ändert,
- eine Applikatorrolle (60) bereitgestellt wird, die teilweise in den Vorrat des fluidischen Materials (66) untergetaucht ist und die so gedreht wird, daß das fluidische Material (66) auf der Oberfläche der Applikatorrolle (60) abgeschieden und davon getragen wird,
dadurch gekennzeichnet,
- daß wenigstens eine Abdeckblende (69) vorgesehen wird, die über der Applikatorrolle (60) angeordnet ist, und
- daß die Bahn (W) in Kontakt mit der Abdeckblende (69) und einem nicht untergetauchten Abschnitt der Applikatorrolle (60), auf dem sich fluidisches Material (66) befindet, bewegt wird, so daß die Bahn (W) nur jene Bereiche (C) der Applikatorrolle (60) kontaktiert, die nicht von der Abdeckblende (69) abgedeckt sind, um fluidisches Material (66) von der Applikatorrolle (60) aufzunehmen.

2. Verfahren nach Anspruch 1, dadurch gekennzeichnet, daß die Lineargeschwindigkeit der Bahn (W) nicht mehr als 10 Prozent der Umfangsgeschwindigkeit der Applikatorrolle (60) beträgt.

3. Verfahren nach Anspruch 1 oder 2, dadurch gekennzeichnet, daß die Bahn (W) aus einem hydrophoben Material hergestellt ist und das fluidische Material (66) so beschaffen ist, daß es die Eigenschaft des Bahnmaterials in einem Bereich (C), in welchem die Bahn die Applikatorrolle (60) kontaktiert, von hydrophob in hydrophil ändert.

4. Verfahren nach Anspruch 3, dadurch gekennzeichnet, daß die Bahn (W) vor dem Aufbringen des fluidischen Materials (66) spinngebunden, schmelzgeblasen, thermogebunden oder klebstoffgebunden wird.

5. Vorrichtung zum Beschichten einer Bahn aus integriertem Material, insbesondere zur Durchführung des Verfahrens nach einem der Ansprüche 1 bis 4
- mit einer Applikatorrolle (60),
- mit einer Masse aus fluidischem Material (66) und
- mit Einrichtungen zum Drehen der Applikatorrolle (60), die teilweise in die Masse aus fluidischem Material (66) untergetaucht ist, um dadurch die Oberfläche der Applikatorrolle (60) mit fluidischem Material (66) zu überziehen,
gekennzeichnet
- durch wenigstens eine Abdeckblende (69), die eine obere Oberfläche hat und die über der nicht untergetauchten Oberfläche der Applikatorrolle (60) angeordnet ist, und
- durch Einrichtungen zum Führen der Bahn (W) über die obere Oberfläche der Abdeckblende (69) und in Kontakt mit dem nicht untergetauchten Abschnitt der Applikatorrolle (60), der nicht von der Abdeckblende (69) abgedeckt ist, wodurch fluidisches Material (66) nur auf den Abschnitt (C) der Bahn (W) aufgebracht wird, der keine Abdeckblende (69) über der Applikatorrolle (60) aufweist.

6. Vorrichtung nach Anspruch 5, gekennzeichnet durch eine angrenzend an die Applikatorrolle (60) angeordnete Spannrolle (61) zum Steuern der Zugspannung in der Bahn (W) sowie zum Steuern der Kontaktfläche (C) der Bahn (W) mit der Rolle (61) und der Applikatorrolle (60).

7. Vorrichtung nach Anspruch 5 oder 6, gekennzeichnet durch Einrichtungen zum Trocknen der Bahn (W) und des fluidischen Materials (66) nach dem Austritt aus der Applikatorrolle (60).

8. Vorrichtung nach einem der Ansprüche 5 bis 7, gekennzeichnet durch Einrichtungen zum Steuern der Geschwindigkeit der Bahn (W) und der Drehzahl der Applikatorrolle (60) derart, daß die Lineargeschwindigkeit der Bewegung der Bahn (W) nicht die gleiche ist wie die Umfangsgeschwindigkeit der Oberfläche der Applikatorrolle (60).

9. Vorrichtung nach Anspruch 8, dadurch gekennzeichnet, daß die Geschwindigkeitssteuereinrichtungen gewährleisten, daß die Umfangsgeschwindigkeit der Applikatorrolle (60) wenigstens zehnmal größer als die lineare Geschwindigkeit der Bahn (W) ist.

## Revendications

1. Procédé pour la fabrication d'une bande pour servir de feuille de couverture dans la production de produits sanitaires absorbants jetables tels que des couches de bébé jetables, des serviettes hygiéniques, des couches pour incontinents, et des compresses pour lits pour hôpitaux, comprenant :
- la fourniture d'une bande (W) de matériau préformé intégré tissé ou non tissé,
- la fourniture d'une source de matériau fluide (66), limitée à une substance qui modifie la propriété de transmission du fluide de la bande (W),
- la fourniture d'un rouleau applicateur (60) partiellement immergé dans ladite source dudit matériau fluide (66) et la rotation dudit rouleau applicateur (60) de telle sorte que le matériau fluide (66) soit déposé sur la surface dudit rouleau applicateur (60) et soit ainsi entraîné à mesure que la bande (W) tourne,
caractérisé par
- la fourniture d'au moins un volet (69) qui est positionné au-dessus dudit rouleau applicateur (60), et
- le déplacement de ladite bande (W) en contact avec ledit volet (69) et une partie non immergée dudit rouleau applicateur (60) sur lequel se trouve un matériau fluide (66) afin que ladite bande (W) soit uniquement en contact avec les zones (C) du rouleau applicateur (60) non recouvertes par ledit volet (69) pour saisir le matériau fluide (66) du rouleau applicateur (60).

2. Procédé selon la revendication 1, caractérisé en ce que la vitesse linéaire de ladite bande (W) n'est pas supérieure à 10 % de la vitesse périphérique dudit rouleau applicateur (60).

3. Procédé selon la revendication 1 ou 2, caractérisé en ce que la bande (W) est fabriquée en matériau hydrophobe et le matériau fluide (66) est tel qu'il modifie la propriété du matériau de la bande, dans une zone (C) où la bande (W) est en contact avec le rouleau applicateur (60), de l'état hydrophobe à l'état hydrophile.

4. Procédé selon la revendication 3, caractérisé en ce que la bande (W) est non tissée, fondue-soufflée, liée thermiquement ou liée par collage avant application du matériau fluide (66).

5. Appareil pour revêtir une bande de matériau intégré, en particulier pour réaliser la méthode selon l'une quelconque des revendications 1 à 4, comprenant
- un rouleau applicateur (60),
- un corps de matériau fluide (66), et
- un moyen pour faire tourner ledit rouleau applicateur (60) partiellement immergé dans ledit corps de matériau fluide (66) de manière à revêtir ladite surface dudit rouleau applicateur (60) avec ledit matériau fluide (66),
caractérisé par
- au moins un volet (69) ayant une surface supérieure et positionné au-dessus de la surface non immergée dudit rouleau applicateur (60) et
- un moyen pour guider ladite bande (W) à travers la surface supérieure du volet (69) et en contact avec la partie non immergée dudit rouleau applicateur (60) non recouverte par ledit volet (69), par quoi ledit matériau fluide (66) est appliqué uniquement à la partie (C) de la bande (W) n'ayant pas un volet (69) au-dessus du rouleau applicateur (60).

6. Appareil selon la revendication 5, caractérisé par un rouleau tendeur (61) adjacent audit rouleau applicateur (60) pour contrôler la tension dans la bande (W) ainsi que pour contrôler la zone de contact (C) de ladite bande (W) avec ledit rouleau (61) et ledit rouleau applicateur (60).

7. Appareil selon la revendication 5 ou 6, caractérisé par un moyen pour sécher la bande (W) et le matériau fluide (66) après avoir passé le rouleau applicateur (60).

8. Appareil selon l'une quelconque des revendications 5 à 7, caractérisé par un moyen pour commander la vitesse de la bande (W) et la vitesse de rotation du rouleau applicateur (60) afin que la vitesse linéaire de déplacement de la bande (W) ne soit pas la même que la vitesse périphérique de la surface du rouleau applicateur (60).

9. Appareil selon la revendication 8, caractérisé en ce que le moyen de commande de la vitesse assure que la vitesse périphérique du rouleau applicateur (60) est au moins 10 fois supérieure à la vitesse linéaire de la bande (W).
